# EUROPEAN PATENT APPLICATION

(11) **EP 1 795 201 A1**
(43) Date of publication of application: **13.06.2007**
(21) Application number: 06386039.9
(22) Date of filing: 04.12.2006
(51) Int. Cl.: A61K 31/7048, C07H 17/04, A23L 1/30, B01D 11/00, A61K 36/63

(54) **Isolation of oleuropein from the leaves of olive tree**

(30) Priority: 09.12.2005 GR 2005100601
(71) Applicant: Mediterrenean Agronomic Institute of Chania (MAICH), 73100 Chania (GR)
(72) Inventor: Kefalas, Panagiotis, 73100 Chania (GR)

(57) **Abstract**

The present invention refers to a new, simple and fast method for the recovery of pure oleuropein after extraction of leaves of the olive tree *(Olea europea).* The method consists in the drying, grinding and extraction of olive tree leaves with a dilute alkaline aqueous solution at a low temperature. The aqueous phase is subsequently extracted with an organic solvent and the resulting solution is concentrated to dryness under vacuum. The residue is further purified by fast column chromatography yielding high purity oleuropein.

## Description

The present invention refers to a new and fast method for the isolation of high purity oleuropein from the leaves of the olive tree. Oleuropein is a compound that has been identified in the leaves of *Olea europaea,* as well as in the bark and roots of the tree. It is a bitter glycoside that decomposes easily, by acid or enzymic hydrolysis, to elenolic acid and glucose. Its use in medicine dates back to the early 19^{th} century, when the leaf extract was used against malaria. To date a plethora of interesting biological properties has been reported; precisely it has been found that it is an efficient antiobiotic *(*Journal Applied Bacteriology 74, 253-259, 1993), antimicrobial and antifungal *(*Microbios 93, 43-54, 1998; J. Appl. Bacteriol. 79, 393-398, 1995) especially against salmonella *(*Letters of Applied Microbiolog 20, 120-124, 1994; Journal of Applied Microbiology 84, 981-987, 1998**)**, food antioxidant *(*Food Chemistry 47, 3531-3534, 1999**;** Eur. J. Lipid Sci. Technol. 107, 497-504, 2005; Phytotherapy Research 12, S98-S100, 1998) and antihypertensive *(*Gazzetta Chimica Italiana 90, 1449-1485, 1960**;** Journal de Pharmacie de Belgique 49, 101-108, 1994).

Many procedures for the recovery of leaf extracts rich in oleuropein have appeared in the literature; however they do not yield high purity product. For example, extraction by hot water followed by warm acetone has been reported, but the the final purity of the product is not mentioned (Gazzetta Chimica Italiana 90, 1449-1485, 1960). Extraction with hydroethanolic mixtures from 25 to 70% ethanol at 20 up to 88 στoυ 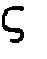 20°C and drying gives a product of purity of 30-40% (US Patent 5,714,150 (1998**)**; WO 99/38383 (1999). Extraction with methanol and liquid nitrogen followed by exclusion of chlorophyll by petroleum ether, purification on XAD resin and silica gel columns *(*Phytochemical Analysis 10, 299-306, 1999). Strong basic environment induces hydrolysis of oleuropein and this is a method for the production of hydroxytyrosol *(*J. Agric Food Chem 44, 2101-2105, 1996; European Journal of Lipid Science and Technology 105(5), 229-242, 2003), while extraction with 95% methanol, aseptic filtration in the presence of water and lyophilisation yields product up to 18% purity (European Patent 1 157 701, 2001).

The present invention aims at the improvement of leaf extraction and the recovery of a product with a high oleuropein content, with a simple and fasr method.

According to the present invention the leaves have been dried prior to use. Drying takes place at room temperature and to the exclusion of light. Then they are ground and the particles of the resulting powder have diameters of 0.05 to 1.00 mm. The ground leaves are further extracted with a weak basic solution at pH8 either at room temperature during 3 to 6 hours or at a higher temperature (35-50 °C) for half to one hour. If necessary the extraction procedure may be repeated once more. The solid residue is filtered and the aqueous phase is subsequently extracted with an organic solvent, as for example diethyl ether, methylene chloride, ethyl acetate etc. This way oleuropein is recovered, while a large amount of water soluble compounds remains in the aqueous phase. The organic phase is then dried on a drying agent like for example anhydrous magnesium sulphate, anhydrous sodium sulphate etc. and the solvent is evaporated to dryness under water pump vacuum (10-20 mmHg) and at a temperature that does not exceed 45 °C. A syrupy product is thus recuperated, which is chlorophyll free and enriched in oleuropein (content 40-50%). This product may be further purified on silica gel flash column chromatography using a mixture of methylene chloride and methanol and water (CH₂Cl₂ : CH₃OH : H₂O) in a ratio of 14:3:1 or 7:3:1. Oleuropein is thus recovered as an amorphous powder with a purity of 95-98% as measured by the HPLC against an external standard.

The advantage of the present invention lies in the fact that the promptly pre treated leaves are fast and easily extracted by a dilute alkaline solution, which is further extracted by an organic solvent that recovers oleuropein selectively. At last, a flash column chromatography with simple organic solvents gives high purity product.

### EXAMPLE

100g of dried and ground olive tree leaves in powder form are mixed with 500 ml 0,1M sodium bisulphate (pH 8.0) and the mixture is stirred at 45 °C for 30 min. The solids are paper filtered and the aqueous phase is extracted with 3 x 200ml ethyl acetate. The organic phase is dried on anhydrous sodium sulphate and concentrated under vacuum at 35 °C. 1,6g of a syrupy product are obtained at a 48% oleuropein content (measurement by HPLC against external standard). This product is further purified by silica gel flash column chromatography, using an appropriate eluant like for example methylene chloride: methanol: water / 14:3:1 or 7:3:1 to yield 0.5 to 1.0 g of oleuroepin as an amorphous powder at a 95-98% purity level.

## Claims

1. A simple and fast method for the recovery of pure oleuropein after extraction of leaves of the olive tree (*Olea europea*), which consists of drying and grinding the leaves and extracting them by a weakly alkaline solution at low temperature. The latter is further extracted with an organic solvent, the residue of which upon concentration and flash column chromatography yields high purity oleuropein.

2. A simple and fast method for the recovery of pure oleuropein according to claim 1, during which the leaves are dried at room temperature to the exclusion of light.

3. A simple and fast method for the recovery of pure oleuropein according to claims 1 and 2, during which the leaves are ground in particles bearing diameters from 0.05 to 1 mm.

4. A simple and fast method for the recovery of pure oleuropein according to claims 1, 2 and 3, during which the ground leaves are extracted with an aqueous alkaline solution at pH 8.0.

5. A simple and fast method for the recovery of pure oleuropein according to claims 1, 2, 3 and 4 during which the aqueous leaf extract is further re-extracted with an organic solvent that selectively recovers oleuropein. Such a solvent maybe diethyl ether, methylene chloride, ethyl acetate etc.

6. A simple and fast method for the recovery of pure oleuropein according to claim 5, during which the evaporation of the organic solvent under vacuum at temperatures that do not exceed 45°C, yields an extract with an oleuropein content ranging from 40-50%.

7. A simple and fast method for the recovery of pure oleuropein according to claim 6, during which the extract is further purified by silica gel flash column chromatography and the use of appropriate elution systems.

8. A simple and fast method for the recovery of pure oleuropein according to claims 5, 6 and 7 during which after selection of the fractions containing oleuropein, concentration of the solvent yields final product of 95-98% purity (determined by HPLC) in the form of amorphous solid.
